# EUROPEAN PATENT SPECIFICATION

(11) **EP 0 483 138 B1**
(45) Date of publication and mention of the grant of the patent: **02.03.1994**
(21) Application number: 89909843.8
(22) Date of filing: 29.08.1989
(51) Int. Cl.: A61B 17/22

(54) **LITHOTRIPTER WITH TWO LOCALIZATION DEVICES IN AN ISOCENTRIC SYSTEM**
LITHOTRIPTER MIT ZWEI ORTSBESTIMMUNGSEINRICHTUNGEN IN EINEM ISOZENTRISCHEN SYSTEM
LITHOTRITEUR AVEC DEUX DISPOSITIFS DE LOCALISATION PLACES DANS UN SYSTEME ISOCENTRIQUE

(30) Priority: 16.11.1988 IT 1517788
(43) Date of publication of application: 06.05.1992
(73) Proprietor: MEDAS SOCIETA PER AZIONI, I-16145 Genoa (IT)
(72) Inventor: PUPPO, Paolo, I-16030 Pieve Ligure (IT)
(74) Representative: Maritano Maello, Giovanna
(86) International application number: IT8900060
(87) International publication number: WO9005492

(56) References cited:
- EP-A- 0 168 559
- EP-A- 0 169 311
- DE-U- 8 515 656

## Description

This present invention concerns an assembled lithotripter with two localization devices for calculi, that are movably arranged about an isocenter and interfaced with a computer, said localization devices for calculi consisting of a radiological device and of an echograph device. The lithotripter and the radiological device are mounted on supporting means for simultaneous unified movement thereof about the isocenter, and the echograph device is freely movable.

According to the present knowledge of the technique, lithotripters are machines that generate shock waves, which are focused on a calculus through a semiellipsoid reflector, causing the fragmentation of the calculus in a line of progression that begins at the surface of the shockwave entry and ends at the point of shock wave exit.

According the the present knowledge of the technique, the position of the calculus is determined with the aid of a radiological device or by echography. Subsequently, the lithotripter is used for the fragmentation.

In a known system, from patent EP-A-0 168 559, the lithotripter is used with two localization devices for calculi, interfaced with a computer, in an isocentric system. In the described lithotripter, once all the devices have been positioned, the isocenter is only verified once for each single operatio. Therefore it is quite a noticeable inconvenience of patent EP-A-0 168 559, for the fact that in each operation, it requires to re-center the device on the calculus, as for example when one wishes to hit the sample calculus from different angles.

An object of this present invention is to be able to isocentricly vary the direction of the shock waves of the lithotripter, changing its delivery angle in the desired direction, without having the calculi move from the position defined by the focus of the ellipsoid, and without the need to move the patient.

These and other objects that will appear in more detail later on, have been achieved according to the present invention, by an assembled lithotripter with two localization devices for the calculi, interfaced with a computer in an isocentric system.

For the purpose of attaining these objects described and to achieve a system in which the devices always remain pointed towards the calculus, from whichever angle these act, the lithotripter and one of the localization devices for the calculi, in this particular instance the radiological device, have been mounted on radially moving slides attached to a component in the shape of a ring. In this manner, their longitudinal axes lie in the same diametric vertical plane, and pass through the center of the supporting ring. The angular distance between the longitudinal axes of the two devices is fixed, as determined by their mounting on the ring. The echograph device, instead, is freely movable and is interfaced with a computer, where the origin of the coordinate system is employed in the computer's calculations and corresponds to the center of the said supporting ring.

Consequently, with this present invention a device is obtained which, on the whole, is far more efficient. It may also be useful for other types of operations, for example endourological, or traditional diagnostic procedures, which can be performed simultaneously, without the need to move the patient.

These and other features and advantages of the present invention will become apparent with reference to the accompanied drawings, that are enclosed only for illustrative purposes and not restrictive , in which:
Fig. 1 illustrates a drawing of the assembly of a lithotripter with localization devices for calculi, according to the present invention:
Fig. 2 illustrates an assembly of a lithotripter with localization devices for calculi according to fig. 1, in a rotated position of 90°:
Fig. 3 illustrates an assembly of a lithotripter with localization devices for calculi according to figure 1, in a rotated position of 180°;
Fig. 4 is the schematic representation of how a calculus can be reached from two different directions;
Fig. 5 illustrates how easily the calculi can be fragmented, with a lithotripter according to present invention;
In the different drawings the same numerals are used for the corresponding parts.

Referring to the drawings, a radiological tube (11), is mounted on the supporting ring (10), with an associated luminance amplifier (12), which is connected to a computer not shown in the drawing. In the middle position between the radiological tube (11), and the luminance amplifier (12), a lithotripter (13) is mounted. The patient's body (14), is positioned on the bed (15), and is introduced inside the supporting ring.

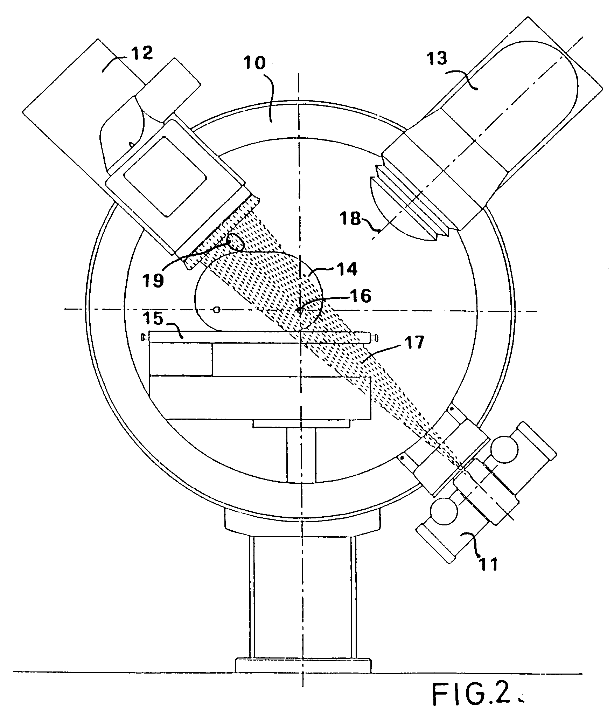

The bed (15) on which the patient's body tests and the ring (10) supporting the two components, known as the radiological tube (11) and the luminance amplifier (12), are moved until the calculus (16) is positioned exactly at the center of the supporting ring (10), and lies in the zone that is created by the rays (17), (Fig. 2).

The radiological device (11 and 12) provides the computer with the x, y, and z coordinates of the calculus to be treated.

The same coordinates can be provided by an echograph, (whose monitor is not illustrated), by means of a free floating probe (19). The echograph is connected to a computer, which calculates the position of the calculus and provides the required table position (x, y, and z as above) which will cause the position of the calculus to coincide with the center of the supporting ring (10).

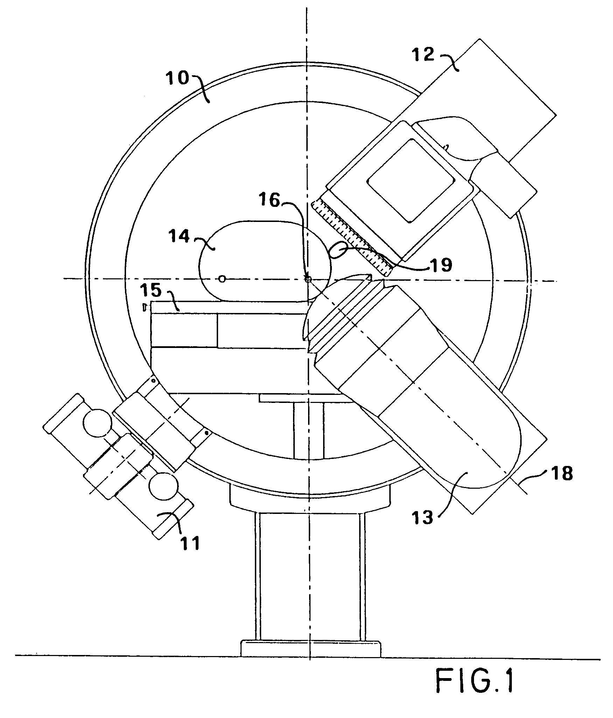

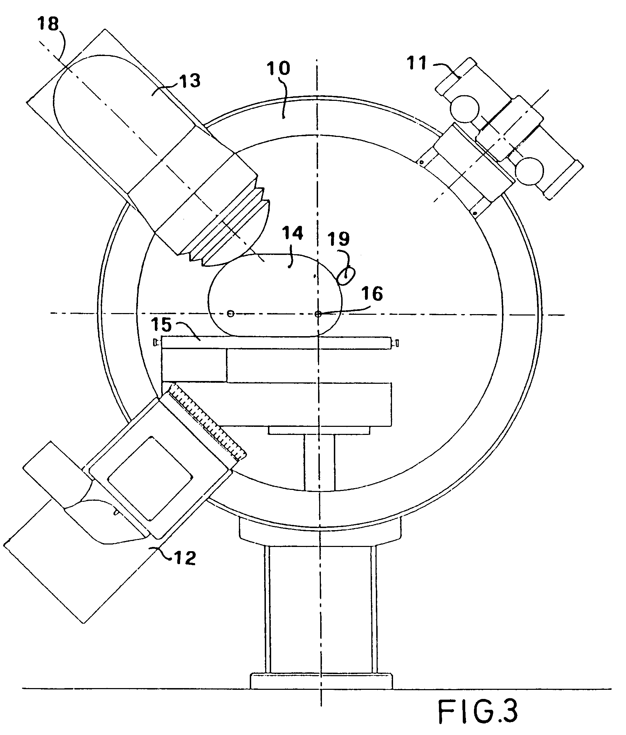

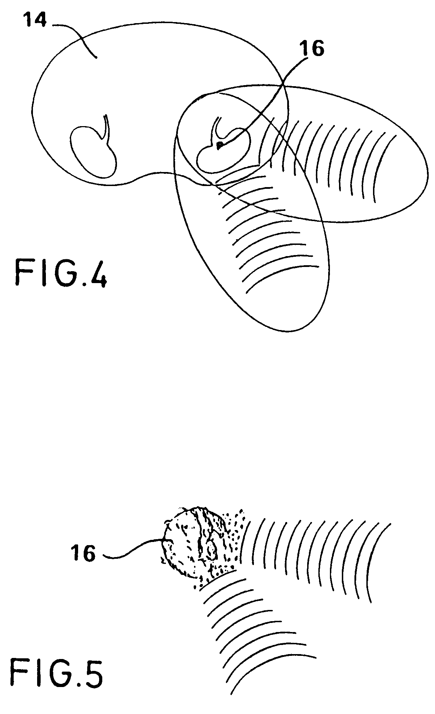

The lithotripter (13), lies on the same plane as the radiological device (11 and 12), and points directly to the center of the ring (10), in which the lithotripter is mounted at a fixed angle to the direction of the ray's axis (17), and can be shifted along it own radial axis (18), to bring is as close as necessary to the patient's body (14), in order to focus the shock waves on the calculus to be fragmented (Fig. 1 and 3), which have been identified by the computer, by radiological means, or by echographical ones. Once the calculus is positioned at the center of the devices, should one verify that another solution would be preferable, with respect to the one chosen originally, and that eventually it would be more expedient to fragment the calculi with waves that originate from different directions, as illustrated in Fig. 4, the supporting ring (10), and the lithotripter (13), may be rotated to the desired position without any need to move the patient, since once localized by the current invention the calculus will always remain centered.

This is especially useful in the case of big calculi, whose fragmentation is easier when the delivery angle of the waves comes from different directions (Fig. 5).

Figures 1, 2, and 3 show three different positions of the supporting ring (10), that carry the radiological device (11), and the lithotripter (13). These drawings show various delivery angles, to illustrate the versatility of the assembly of the components according to the present invention.

The lithotripter (13) and the radiological device (11) can be simultaneously rotated in a range of approximately, 260°, remaining centered on the same focus.

Maximum precision and minimal trauma to the patient results from the precise localization of the calculus, the isocentric nature of the system, and the subsequent visualization of the fragmentation process in real time. Due to the simultaneous use of the radiological and echographic devices, the isocentric system, according to the present invention, is particularly versatile, and usable for any type of localization and calculi.

According to a preferred embodiment of the invention, the size of the minor diameter, of the semiellipsoidal reflector used to focus the shock waves is greater than that of other known lithotripters, which decreases the algogenic stimuli caused by the shock waves on the skin; this is very beneficial in the range of about 30 cm.

With the lithotripter mounted on a supporting ring, maintenance and electrode changing becomes more efficient in respect to other known lithotripters due to the accessibility of the lithotripter on this present invention.

## Claims

1. Lithotripter apparatus comprising a lithotripter (13) and two localization devices for calculi interfaced with a computer, said lithotripter (13) and said two localization devices being movably arranged about an isocenter, said localization devices for calculi consisting of a radiological device (11-12) and of an echograph device (19), characterised in that the lithotripter (13) and the radiological device (11-12) are mounted on supporting means for simultaneous unified movement thereof about the isocenter, and the echograph device (19) is freely movable.

2. Lithotripter apparatus as set forth in claim 1, wherein the lithotripter (13) and the radiological device (11-12) are mounted on radially movable slides attached to a supporting ring (10), and the lithotripter (13) and the radiological device (11-12) are placed on said ring with their axis on the same diametrical vertical plane at a fixed angle relative to each other, the center of the said supporting ring corresponding to the origin of the coordinate system, which is employed in the computer's calculations, in conjunction with the use of an echograph device.

## Patentansprüche

1. Lithotripsiegerät bestehend aus einem Lithotripter (13) und aus zwei mit einem Computer verbunden Steinlokalisierungsgeräten , dieser Lithotripter (13) und diese zwei Lokalisierungsgeräte sind über ein Isozentrum beweglich angebracht,
die besagten Steinlokalisierungsgeräte bestehen aus einem Röntgenapparat (11-12) und aus einem Echographen (19), kennzeichnend ist, daß der Lithotripter (13) und der Röntgenapparat (11-12) für simultan vereinte Bewegung über das Isozentrum aus Stützeinrichtungen montiert sind und der Echograph (19) frei beweglich ist.

2. Lithotripsiegerät, wie unter Anspruch 1 angegeben, wobei der Lithotripter (13) und der Röntgenapparat (11 -12) auf radial bewegliche, an einen Unterstützungsring (10) angebrachte Schienen montiert sind, der Lithotripter (13) und der Röntgenapparat (11-12) sind an dem besagten Ring mit ihrer Achse auf der gleichen vertikalen diametralen Ebene perpendikulär einander gegenüber liegend angebracht, das Zentrum des besagten Unterstützungsringes entspricht dem Ursprung des Koordinatensystems, das in den Computerberechnungen angewandt wird, in Verbindung mit dem Gebrauch eines Echographen.

## Revendications

1. Appareil lithotriteur comprenant un lithotriteur (13) et deux dispositifs pour la localisation de calculs interfacés avec un ordinateur, où ce lithotriteur et ces dispositifs de localisation sont aménagés de façon mobile autour d'un isocentre, ces dispositifs de localisation des calculs étant formés par un appareil radiologique (11-12) et un échographe (19), caractérisé par le fait que le lithotriteur (13) et l'appareil radiologique (11-12) sont montés sur un support pour assurer leur mouvement unifié et simultané autour d'un isocentre et que l'échographe (19) est librement mobile.

2. Appareil lithotriteur comme indiqué dans la revendication 1, caractérisé par le fait que le lithotriteur (13) et l'appareil radiologique (11-12) sont montés sur des guides mobiles en sens radial, attachés à une bague de support (10) et que le lithotriteur (13) et l'appareil radiologique (11-12) sont montés sur cette bague avec leur axe dans le même plan diamétral à un angle fixe, l'un relatif à l'autre et que le centre de cette bague de support correspond à l'origine du système de coordonnées utilisé par l'ordinateur pour le calcul en conjonction avec l'emploi de l'echographe.
